Europäisches Patentamt

European Patent Office

Office européen des brevets

(19)

(11) Publication number: **0 359 714 B1**

# EUROPEAN PATENT SPECIFICATION

(12)

(45) Date of publication of patent specification: **12.01.94**

(51) Int. Cl.⁵: **C12N 15/16**, C07K 7/10, C12N 15/86, C12P 21/02, C12N 7/00, A01N 63/00

(21) Application number: **89810675.2**

(22) Date of filing: **12.09.89**

(54) **Diuretic factor.**

(30) Priority: **16.09.88 US 245429**

(43) Date of publication of application:
**21.03.90 Bulletin 90/12**

(45) Publication of the grant of the patent:
**12.01.94 Bulletin 94/02**

(84) Designated Contracting States:
**BE CH DE FR GB IT LI NL**

(56) References cited:
**EP-A- 0 127 839**

**PROC. NATL. ACAD. SCI. USA, vol. 86, April 1989, pages 2976-2980, Washington,DC, US; H. KATAOKA et al.: "Isolation and identification of a diuretic hormonefrom the tobacco hornworm, Manduca sexta"**

**CHEMICAL ABSTRACTS, vol. 101, 1984, page 430, abstract no. 148309g, Columbus,Ohio, US; P.J. MORGAN et al.: "Diuretic hormone: another peptide withwidespread distribution within the insect CNS", & PHYSIOL. EN-TOMOL: 1984,**

(73) Proprietor: **SANDOZ AG**
**Lichtstrasse 35**
**CH-4002 Basel(CH)**

(84) Designated Contracting States:
**BE CH FR GB IT LI NL**

(73) Proprietor: **SANDOZ-PATENT-GMBH**
**Humboldtstrasse 3**
**D-79539 Lörrach(DE)**

(84) Designated Contracting States:
**DE**

(72) Inventor: **Kataoka, Hiroshi**
**Mukougaoka 2-35-8-302**
**Bunkyo-Ku**
**Tokyo 113(JP)**
Inventor: **Kramer, Steven Jan**
**858 Hollenbeck Avenue**
**Sunnyvale, CA 94087(US)**
Inventor: **Maeda, Susumu**
**700 Elmwood Drive**
**Cadiv, CA 95616(US)**
Inventor: **Roter, Alan Harris**
**755 B Loma Verde Avenue**
**Palo Alto, CA 94303(US)**

9(2), 197-206

CHEMICAL ABSTRACTS, vol. 109, 1988, page 403, abstract no. 4208r, Columbus,Ohio, US; G.D. WHEELOCK et al.: "Evidence for hormonal control of diuresisafter a blood meal in the mosquito Aedes aegypti",& ARCH. INSECT BIOCHEM. PHYSIOL. 1988, 7(2), 75-89

SCIENCE, vol. 219, February 1983, pages 715-721, Lancaster, GB; L.K. MILLER etal.: "Bacterial, viral, and fungal insecticides"

Inventor: **Schooley, David Allan**
**3024 Greer Road**
**Palo Alto, CA 94303(US)**
Inventor: **Troetschler, Ruth Gene**
**184 Lockhart Lane**
**Los Altos, CA 94022(US)**

## Description

The present invention relates to the isolation and characterization of an insect diuretic factor. The isolated diuretic factor (DF) was a C-terminus amide polypeptide which directly or indirectly affects fluid secretion in the insect. Means for recombinant production and utilization of C-terminus polypeptides having insect diuretic factor activity are also provided.

Brief Description of the Invention

Diuretic factor (DF) was extracted from heads of the tobacco hornworm Manduca sexta and then purified using a multi-step procedure involving ion exchange column chromatography, reversed-phase chromatography, and liquid chromatography (semi-preparative, cation-exchange, analytical, and microbore). The product was a substantially homogenous polypeptide.

After purification, the DF polypeptide was analyzed and its amino acid sequence was determined. The native peptide was found to involve a sequence of 41 amino acids having a C-terminal amide.

While not wishing to be bound by theory, it appears that the DF according to this invention may be the same as diuretic hormone. On the other hand, since the DF peptide of this invention shows some amino acid sequence homology to certain hormone releasing factors such as corticotropin releasing factors, it is contemplated that the DF peptide of the present invention may act by stimulating the release of diuretic hormone. In either case, the same biological end result, i.e. regulation of insect fluid balance is achieved.

Synthetically produced or isolated DF may be used as insecticides and insect population control agents by directly or indirectly affecting the normal fluid balance in the insect.

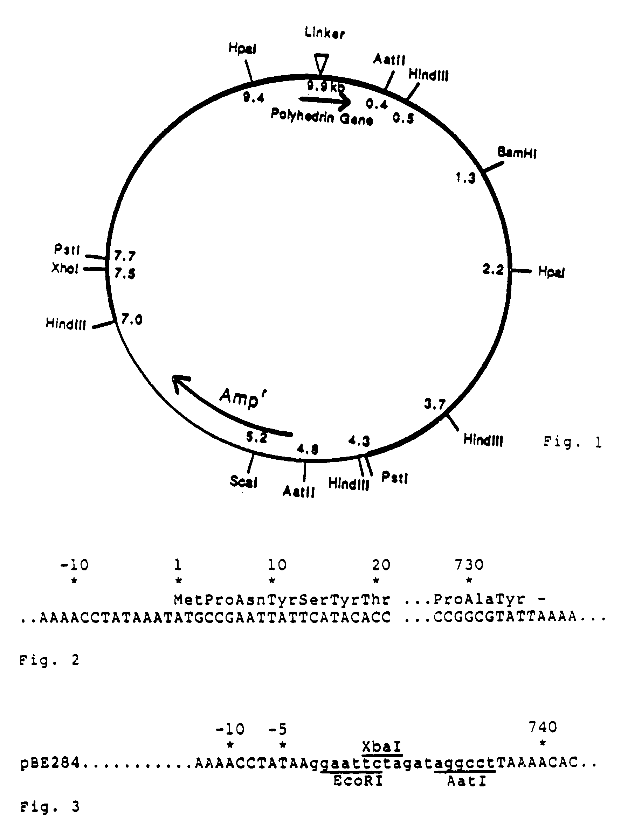

In the accompanying drawings, Figures 1, 2, 3 and 4 represent intermediate plasmids in a making of hybrid baculoviruses.

The following definitions will apply throughout the specification and claims:

Diuretic factor activity (also herein diuretic factor-like activity) - the activity of a polypeptide observed in the in vivo M. sexta assay described herein (below and in Example A) which mimics or evokes substantially the same type of diuretic response as native diuretic factor from Manduca sexta in said assay (exhibited by diuretic factor, herein DF, or a diuretic factor analog, herein DFA, as described herein).

Native diuretic factor - a polypeptide which is found in certain insects which is directly or indirectly involved in the regulation of fluid balance.

Associated insect polypeptides - polypeptides occurring naturally in an insect which are other than DF.

Detailed Description of the Invention

One aspect of this invention provides for a polypeptide having diuretic factor-like activity which is substantially free from associated insect polypeptides and having the following amino acid sequence and an amidated C-terminus according to Formula I:

```
Arg-Met-Pro-Ser-Leu-Ser-Ile-Asp-Leu-Pro-Met-Ser-Val-
Leu-Arg-Gln-Lys-Leu-Ser-Leu-Glu-Lys-Glu-Arg-Lys-Val-His-
Ala-Leu-Arg-Ala-Ala-Ala-Asn-Arg-Asn-Phe-Leu-Asn-Asp-Ile-
NH2                                            (Formula I)
```

The above peptide with the amidated C-terminus is presumably the result of an enzymatic hydrolysis of a peptide whose terminus is or contains glycine. Thus, another aspect of this invention provides for a precursor peptide having the following amino acid sequence according to Formula II:

```
Arg-Met-Pro-Ser-Leu-Ser-Ile-Asp-Leu-Pro-Met-Ser-Val
Leu-Arg-Gln-Lys-Leu-Ser-Leu-Glu-Lys-Glu-Arg-Lys-Val-His-
Ala-Leu-Ala-Ala-Ala-Asn-Arg-Asn-Phe-Leu-Asn-Asp-Ile-X
```

                                                            (Formula II)

wherein X is Gly, Gly-Lys or Gly-Lys-Arg, preferably Gly.

The native DF obtained as described hereinafter was digested with trypsin, and six fragments were generated. The fragments were sequenced and are shown below.

| Fragment | Position | Sequence |
|----------|----------|----------|
| T-6 | 2-15 | Met-Pro-Ser-Leu-Ser-Ile-Asp |
| | | Leu-Pro-Met-Ser-Val-Leu-Arg |
| T-4 | 18-22 | Leu-Ser-Leu-Glu-Lys |
| T-3 | 25-30 | Lys-Val-His-Ala-Leu-Arg |
| T-2 | 26-30 | Val-His-Ala-Leu-Arg |
| T-1 | 31-35 | Ala-Ala-Ala-Asn-Arg |
| T-5 | 36-41 | Asn-Phe-Leu-Asn-Asp-Ile |

Since T-5 had neither Arg nor Lys at its carboxyl terminus, fragment T-5 is the carboxyl terminus of the polypeptide.

The nature of the carboxyl terminus was established by comparing the reversed-phase liquid chromatography retention times of T-5 and two synthetic peptides (Asn-Phe-Leu-Asn-Asp-Ile) prepared in the amidated and free acid forms. The results clearly showed that the carboxyl terminus is amidated.

The biological activity of the isolated DF was tested on the butterfly Pieris rapae. To assay on P. rapae, newly emerged adult butterflies were neck-ligated and beheaded, depriving the abdomen of DF which ordinarily stimulates the substantial loss of a clear fluid from the rectum. Neck-ligated butterflies excrete only small amounts of a dark meconium (waste product of pupal metabolism). Injection of DF-active fractions into ligated butterflies caused a typical normal urine excretion.

The effects of M. sexta DF were investigated on M. sexta larvae in vivo. These larvae (late fifth stadium, weighing approximately 9g) excrete approximately one third of their body weight in water over a 48 hour period. Larvae injected with 0.1 $\mu$g of synthetic DF lost over twice as much fluid over a 4 hour test period than did controls which did not receive any DF.

This invention also provides the use of DF and DFA (diuretic factor analogs, hereinafter defined) as insecticides or as agents to enhance the activity of other insecticides. A composition for such purposes comprises an effective amount of DF or DFA combined with an inert carrier which does not adversely effect the active peptide ingredient, but which preferably facilitates contact uptake or absorption of the DF or DFA, such as DMSO and water. Such compositions typically contain 0.1 mg to 1.0 mg DF or DFA polypeptide per ml of inert carrier (when said inert carrier is a liquid), e.g. 0.1 to 1.0 mg DF or DFA polypeptide per ml of 30% aqueous DMSO. The composition may optionally contain suitable adjuvants such as dispersants, e.g. sodium lauryl sulfate or silica, and wetting agents, e.g. Toximul 360A (a mixture of anionic and nonionic surfactants). Typically the adjuvants are present in amounts up to 20% by weight of the composition. This composition is applied to the insect or its environment. Typical dosages range from 0.5-50 kg/hectare, preferably 2-20 kg/hectare, with applications repeated as required.

Also provided by this application are processes for the production of DF or DFA polypeptide. DF and DFA polypeptide may be provided by conventional synthetic methods, e.g. using solid phase synthesis. Alternatively, and more conveniently, DF and DFA polypeptide are produced by biotechnical expression in a recombinant hybrid virus as hereinafter described.

Further provided by the invention are DNA sequences encoding diuretic factor and polypeptides having diuretic factor activity. More particularly, the invention provides DNA sequences encoding such polypeptides which have the C-terminus amide, and especially such DNA when combined with heterologous DNA to form systems for the expression, production and/or delivery of polypeptides which have diuretic factor-like activity (including diuretic factor itself). Such DNA sequences may be routinely constructed from the knowledge of the genetic code with the use of conventional DNA synthesizers. The term "heterologous DNA" refers to DNA not associated with the DNA for the production of diuretic factor polypeptides in nature (or a structural gene for an analog thereof). Heterologous DNA typically involves promoter or operator sequences but may simply involve plasmid or vector DNA not operatively involved in expression.

By way of illustration only, Table A below shows the amino acid sequence of our native diuretic factor isolate and above each amino acid thereof a DNA codon (or formula therefor) is given to provide a DNA sequence encoding such diuretic factor, such DNA sequence terminating in a codon for Gly as an additional amino acid enabling the formation of 57-position Ile-NH$_2$ (amide) when the DNA are expressed in an appropriate system or environment, eg. in insect cells.

Addition of a codon for Gly to the DNA sequence results in expression of a pro-peptide resulting in the mature 41 amino acid active peptide in appropriate cell systems or environment. The 58-position Gly in Table A may be extended (and the required DNA codons provided in the nucleotide sequence) to provide other proforms for C-terminus Ile-NH$_2$ in diuretic factor. For example, DNA sequences encoding for Lys (AAG) and Lys-Arg (AAGCGG) downstream from Gly codon (GGC) have been also indicated in our work to result in a polypeptide having the activity effects of the polypeptide produced from DNA sequences encoding the terminal Gly-Stop.

As will be appreciated, the nucleotide sequence illustrated in Table A is but one of a variety of nucleotide sequences which may be constructed to encode the 41 amino acid sequence of DF and its signal and stop sequences as given in Table A. Hence, different codons encoding the same amino acid in such amino acid sequence may be readily substituted as allowed by the genetic code. All of such codon or nucleotide changes while retaining a sequence coding for the 41 amino acids of the mature DF polypeptide will nevertheless result in a nucleotide or DNA sequence which is highly homologous to the 123 nucleotide sequence set forth in Table A. Such homology may be particularly indicated by the ability of a segment of DNA having 123 bp and corresponding to the 123 nucleotide sequence encoding mature DF as shown in Table A to hybridize under stringent hybridization conditions to the DNA which has a different sequence than the 123 nucleotide sequence shown in Table A but which nevertheless encodes the same 41 amino acids shown in Table A for mature DF.

As will also be appreciated and as contemplated herein, various changes may be made in the 41 amino acid sequence of mature DF as depicted in Table A and by Formula I while still providing a polypeptide having the type of biological activity of the mature DF sequence depicted in Table A. Such modified polypeptides are herein referred to as Diuretic Factor Analogs (DFA) and evoke substantially the same type of diuretic response in the in vivo M. sexta assay described herein as the native mature DF sequence depicted in Table A. Such modifications of the amino acid sequence of the native mature DF depicted in Table A (and Formula I) include deletions of one or more amino acids such as for example those at or near the N-terminus, substitutions of one or more amino acids for another involving for example exchange of homologous natural amino acids or substitutions for amino acids which are not essential for DF activity and additions or extensions by one or more amino acids of the native 41 amino acid sequence such as for example extensions at the N-terminus. DNA sequences having a coding or sense strand encoding such DFA polypeptides may be constructed analogously to the manner in which the DNA for the DF depicted in Table A was constructed as described herein. Such DNA sequences encoding a DFA polypeptide are preferably and particularly those which will also hybridize under stringent hybridization condition to a 123 bp DNA segment having or incorporated in one of the two strands thereof the same 123 nucleotide sequence depicted in Table A as encoding the 41 amino acids of the native mature DF depicted in Table A. Stringent hybridization conditions as contemplated herein are those in which hybridization is effected in a standard manner at 68°C in 6.0 X saline citrate buffer (a.k.a. SSC buffer) followed by merely rinsing at 37°C at reduced buffer concentration (which will not affect the hybridizations which have taken place). The 123 bp DNA to be used for hybridization may be conventionally prepared, for example, by analogy to the procedure for preparing the DF structural gene sequence as described herein, and may be tagged to form an identifiable hybridization probe by procedures well known in the art. Such a probe may also be used to probe suitable and conventionally prepared clone banks to locate and isolate alleles (both nucleotide and amino acid alleles) of the DF of M. sexta or to locate and isolate genes coding for DF in other or related insect species. Selection of particular codons for particular amino acids is preferably in accord with natural preferences for the cell in which expression is desired.

This invention, therefore, further provides a polypeptide having insect diuretic activity which is coded for by a) a DNA sequence comprising the 123 nucleotides extending from nucleotide position number 54 to nucleotide position number 177 in Table A; or b) a nucleotide sequence which hybridizes under stringent conditions to such 123 nucleotide sequence.

A particular method for use of DF or DFA as an insecticide is using an insect-specific virus (baculovirus) as a vector. A gene encoding DF or DFA is inserted into the baculovirus DNA, and is under the control of a baculovirus promoter. After an insect ingests the recombinant bybrid baculovirus DNA, the virus DNA multiplies inside the insect and DF or DFA is expressed (produced) in a sufficient amount as to enhance insecticidal effect of the virus DNA. Such recombinantly modified baculovirus DNA may also be used as vectors to transform or transfect cells, particularly insect cells, to provide systems for the production of DF or DFA.

A number of baculovirus are known in the art, which would be suitable for use as vectors, including Autographa californica Nuclear Polyhedrosis Virus and Bombyx mori Nuclear Polyhedrosis Virus, with Autographa californica being the preferred baculovirus. The codon preferences for the particular virus chosen can be determined by comparing codon usage in known baculovirus sequences. A structural gene for DF or DF is then synthesized adding a codon for C-terminal glycine, or equivalent sequence which will be post-translationally modified to $NH_2$, followed by a stop codon, preferably utilizing these codon preferences. The structural gene is preferably ligated to a signal or secretion sequence of the type to provide for the transport of expressed protein through the cell membrane, such sequence being processable upon secretion to cleave the sequence from the proform to provide the mature product. Many signal sequences are known in the art including the signal sequence associated with larval cuticle protein 2 of the fruit fly Drosophila melanogaster, reported by Snyder et al., 1982 Cell v29 1027-1040.This sequence can be used in its native form, or preferably, can be altered so that its codons conform to those preferred by the baculovirus.

The signal sequence and structural gene are placed downstream from a desired baculovirus promoter. Examples of suitable promoters include the NPV polyhedrin gene promoter. One method of inserting the DF construct is by using a transfer or shuttle vector containing a DF or DFA gene sequence flanked on at lest one and preferably on both sides by a region of baculovirus DNA. A double crossover or cotransfection occurs upon combining the virus DNA and shuttle vector under cotransfection conditions. The result is a recombinant hybrid baculovirus containing an expressible DF or DFA gene.

More particularly, and again by way of illustration only a full length DNA sequence encoding diuretic factor and including a signal sequence, the DF structural gene with terminal Gly codon and stop signal and useful for construction of such baculovirus systems is given below in Table A along with relevant amino acids encoded by such sequence.

## Table A

```
EcoRI
  |                                                                      69
GAATTCATGTTTAAGTTTGTTATGATCCTTGCAGTGGTGGGCGTGGCCACGGCCCGAATGCCGTCTCTA

        METPheLysPheValMETIleLeuAlaValValGlyValAlaThrAlaArgMETProSerLeu
        (1)                                                 (16)(17).         (21)


        BglII                                   XbaI
          |                                       |                      138
TCGATAGATCTGCCCATGAGCGTCCTCAGACAGAAACTTAGTCTAGAAAAGGAGAGAAAAGTTCACGCG

SerIleAspLeuProMETSerValLeuArgGlnLysLeuSerLeuGluLysGluArgLysValHisAla
              (27)                                                          (44)


                                       EcoRV    HindIII
                                         |        |  187
          TTACGTGCAGCTGCCAATAGGAACTTTTTAAACGATATCGGCTAAGCTT

          LeuArgAlaAlaAlaAsnArgAsnPheLeuAsnAspIleGly .
                                 (57) (58)
```

In Table A, nucleotides are numbered above the line in which the nucleotides appear while amino acids are numbered in parenthesis below the line in which the amino acid sequence is given. Certain restriction sites in the nucleotide sequence are also indicated above the nucleotide sequence at about the point where they occur. As will be evident, amino acid positions 1 through 16 represent the signal sequence and positions 17 through 57 represent the amino acid sequence of the nature diuretic factor with the Gly at position 58 provided to form the Ile-NH$_2$ in the final expressed mature product.

The DNA sequence represented by Table A may be conveniently constructed and provided in a conventional manner by forming four DNA sequences from the eight synthesized oligonucleotides (OC and ON, 1C and 1N, 2C and 2N and 3C and 3N) shown in Table B, below.

## Table B

Oligo OC:

5' AATTCATGTTTAAGTTTGTTATGATCCTTGCAGTGGTGGGCGTGGCCACGGCCGGATCCATGCATCCGAGCT 3'

Oligo ON:

5' CGGATGCATGGATCCGGCCGTGGCCACGCCCACCACTGCAAGGATCATAACAAACTTAAACATG 3'

Oligo 1C:

5' CGCATGCGAATGCCGTCTCTATCGATAGATCTGCCCATGAGCGTCCTCAGACAGAAACTTAGT 3'

Oligo 1N:

5' CTAGACTAAGTTTCTGTCTGAGGACGCTCATGGGCAGATCTATCGATAGAGACGGCATTCGCATGCGAGCT 3'

Oligo 2C:

5' CTAGAAAAGGAGAGAAAAGTTCACGCGTTACGTGCAGCTGCCAATAGGAACTTTTTAAACGATATCTA 3'

Oligo 2N:

5' AGCTTAGATATCGTTTAAAAAGTTCCTATTGGCAGCTGCACGTAACGCGTGAACTTTTCTCTCCTTTT 3'

Oligo 3C:

5' ATCGGCTA 3'

Oligo 3N:

5' AGCTTAGCCGAT 3'

As will be evident from Table B, the oligonucleotide sequences OC (72 nucleotides) and ON (64 nucleotides) will hybridize (anneal) to form DNA (herein DNA-O) with 4 nucleotides overhanging each end for effecting ligation. Similarly, the sequences 1C (46 nucleotides) and 1N (71 nucleotides) will anneal to form DNA (herein DNA-1) with 4 nucleotides overhanging each end. Similarly, the sequence 2C (68 nucleotides) and the sequence 2N (68 nucleotides) will anneal to form DNA (herein DNA-2) with 4 nucelotides overhanging each end. Finally, the sequences 3C (8 nucelotides) and 3N (12 nucleotides) will anneal to form DNA (herein DNA-3) with 4 nucleotides overhanging one end. The DNA-O codes for the upstream portion of the diuretic hormone gene sequence of Table A (plus one nucleotide) and DNA-1, DNA-2 and DNA-3 code for successive downstream sections (plus one nucleotide added by DNA-3). The DNA-0, DNA-1, DNA-2 and DNA-3 have complementary overhanging ends enabling them to be joined in a simple ligation fashion to form the entire DNA represented in Table A, and the overhangs at each end are designed to represent the "sticky" end otherwise formed by restricting endonuclease digestion (with EcoRI and Hind III), thereby enabling simple ligation of the constructed gene containing DNA segment with other DNA which have been cut with such endonucleases to form companion overhangs for complementary ligation.

In order to construct a desired intermediate plasmid pDHC, DNA-2 was ligated with the large fragment obtained on digesting the known plasmid pTZ19R (Pharmacia Inc.) with the restriction endonucleases XbaI and HindIII to form the plasmid pDH-1 which was used to transform bacterial cells to obtain copies and confirm structure. The DNA-1 was ligated with the large fragment obtained on digesting the plasmid pDH-1 with the endonucleases SacI and XbaI to form the plasmid pDH-2 which was similarly used to transform bacterial cells. The DNA-O was then ligated with the large fragment obtained on cutting the known plasmid pTZ18R (Pharmacia Inc.) with EcoRI and SacI to form the plasmid pDH-3 and this plasmid then cut with SacI and ScaI and 1.8Kb fragment isolated and ligated with 1.2Kb fragment obtained on cutting pDH-2 with SacI and ScaI, to form plasmid pDH-4 which contains a spacer region approximately located between EagI and SphI. The plasmid pDH-5 is therefore formed by cutting pDH-4 with EagI and SphI, treating with Klenow Fragment to fill in the 5′ overhangs and re-circularizing (ligating the large fragment DNA). The plasmid pDHC is then constructed by ligating the large fragment obtained on cutting (digesting) pDH-5 with EcoR and HindIII with the DNA-3 fragment, followed again by transformation of bacteria to obtain copies and confirm structure.

The diuretic factor gene including the secretion (signal) sequence therefor as available in plasmid pDHC is then excised from pDHC and placed under transcriptional control of polyhedrin gene sequences obtained from baculovirus DNA. A shuttle vector containing the resulting recombinant gene is contacted with baculovirus DNA to effect a recombination or crossover resulting in incorporation of recombinant gene into the baculovirus genome. The resulting modified or hybrid baculovirus DNA is recovered and multiplied and may be used in enhancing the toxic or insecticidal action of the baculovirus in combatting insects. Such recombinant baculovirus may also be used as expression vectors to infect (transform or transfect) insect cells, such as Bombyx mori cells, to produce DF and DFA. The basic method for preparation of such modified viruses is known and described for example in U.S. patent 4,745,051. The ability of the diuretic factor-like polypeptides to be expressed in active form in such systems and/or enhance insecticidal activity is judged unexpected.

Any of the polyhedrin gene containing Baculoviruses (nuclear polyhedrosis viruses or NPV) may be used in preparing such vectors and insecticides, including Autographa Californica NPV as described in detail in U.S. patent 4,745,051. Another system based on Bombyx mori NPV is also of interest and basic information thereon is available, see, for example, published European Patent Application 0175852, and may be employed to demonstrate the use of NPV's for expression of DF and the DFA polypeptides and enhancement of insecticidal activity of the NPV therewith.

By way of illustration only, the Bombyx mori NPV (BmNPV) genome involves circular DNA of about 130 Kb. DNA containing transcriptional control sequences of the polyhedrin gene is excised from the genomic DNA and combined in a transfer (shuttle) vector with the DFA gene preferably such that the start codon (ATG) of the DF gene is 5 bp downstream from where the polyhedrin start codon (ATG) would have been located.

More particularly, the DNA containing the transcriptional control sequences of the polyhedrin gene from BmNPV are incorporated into a plasmid of the type of pBmE36 as described in the above indicated European Patent Application No. 0175852 (see also Maeda et al. Nature, 315:592, 1985). Such a plasmid has about 10.5 Kb and contains the polyhedrin gene in pBR322 sequences, and is represented in Fig. 1 with relevant sequences from the polyhedrin gene as found in pBmE36 given in Fig. 2. The polyhedrin structural gene is excised and a synthesized linker or plasmid polylinker is inserted after the promoter of the polyhedrin gene. Such vectors possess about 3 Kb of each of the 5' and 3' fragments from Bombyx mori DNA for later homologous recombination, a linker between these fragments for insertion of DF gene, a marker gene (for ampicillin) and bacterial origin of replication. In this way, a vector DNA is formed comprising a DNA sequence encoding a DF or DFA polypeptide that is 5' and 3' flanked by an insect virus DNA sequence comprising insect virus polyhedrin structural gene DNA. The plasmid pBmE36 is used to prepare a more preferred vector pBE284 as described hereinafter in Example 8. The vector pBE284 still possesses the 3 Kb flanking fragments from Bombyx mori located before and after the polyhedrin gene and contains the complete 5' flanking region except for two base pairs before the translational start. The vector pBE284 has additional Kpn I and Sac I sites. Fig. 1 shows the basic plasmid of the pBmE36 type with the numbering of nucleotide positions given along with the general location of the polyhedrin gene insect (linker area). Fig. 2 shows in detail the sequence around the translation start codon of the polyhedrin gene (linker area) before deletion of the polyherin structural gene and before the modifications to provide pBE284. Fig. 3 shows the sequence in the linker area in pBE284 along with introduced restriction sites, with the sequences altered with regard to Fig. 2 indicated by lower case letters. Hence, the plasmid pBE284 provides EcoRI, Xba I and AatI restriction sites just downstream from the polyhedrin promoter sequence and thereby a means for introducing the diuretic factor gene into the plasmid. The preparation of the desired shuttle vector, herein pBmDH-1, from pDHC and pBE284 is indicated hereinafter in Example 9.

The shuttle vector pBmDH-1 is then exposed to the viral DNA or BmNPV to transfect the viral DNA with the DF-containing BmNVP sequences from pBmDH-1. The details for such cotransfection to obtain recombinant BmNPV virus capable of expressing the DF gene and forming DF in infected insects for insecticidal use of the recombinant virus is described hereinafter in Example 7.

The DF or DFA polypeptide may be produced biotechnologically from the DF or DFA gene-containing modified baculoviruses of the invention in essentially a known manner. Host cells are cultured (grown) to about the final volume desired for production, then infected with the modified baculovirus DNA of the invention and the resulting infected (transformed or transfected) cells maintained to allow expression (production) of the DF or DFA. Such processes are run on a batch basis in view of the destructive effect of the virus on the cells. Suitable host cells are capable of amidating the C-terminus of an amino acid sequence, and include e.g. insect cells, mamallian cells, and yeast cells.

The modified baculovirus of the invention may be themselves used as insecticides in the general manner in which the unmodified native viruses may be used, i.e. by application of an effective amount to the locus of insects susceptible to the virus. The modified viruses as produced above have been found viable or competent for such purposes by containing native DNA sequences for reproduction, lethal infection and expression of the DF or DFA polypeptide. The modified viruses may be advantageously also combined with and otherwise coapplied with the unmodified, native viruses. Insecticidal compositions containing such modified or native baculorivus in an insecticidally effective amount will typically comprise inert carriers such a clay, lactose and proteinaceous materials such as defatted soybean powder to assist in application, particularly those carriers such as the proteinaceous materials which are feeding attractants and which may enhance stability of the contained baculoviruses. Such compositions are desirably stored at lower temperatures, e.g. minus 20°C, to enhance presentation of the modified and unmodified viruses.

The compositions provided by the invention may be particulate compositions in that the compositions may be composed of a multiplicity of fine particles in which the baculovirus is secured in a clay and/or protein matrix. The term "secured" as used herein in connection with such particles embraces an imbedding in the matrix with some portion of the baculovirus exposed at the surface of the particle and also embraces a complete encapsulation of the baculovirus in the matrix, the latter condition being preferred and also predominant in the particles of the invention. The particles in the composition are advantageously characterized by a fine size not exceeding 150 microns up to 150 microns, and it is a further feature of the invention that satisfactory compositions having particle size of from 5 to 100, preferably 5 to 50 microns and particularly 5 to 25 microns, can be readily produced.

The matrix material constitutes 65 to 99.9 percent by weight of the total weight of the baculovirus and matrix material in this embodiment of the compositions of the invention, preferably 85 % to 99 % by weight. The clay employed in the composition may be any of the commercially available processed clays of a fine, essentially powdered, nature including, by way of illustration, Kaoline clays, Olancha clays, Attapulgus clays and Betonite clays. The preferred clays are Olancha and Attapulsites. The vegetable protein employed as matrix material may also be from any of a wide variety of sources of protein which have been processed into a fine, essentially powdered form. The materials are preferably defatted or otherwise substantially fat-free. Vegetable protein sources which may be mentioned by way of illustration include soybeans, cottonseeds, sunflower seeds and extracts of various yeasts. The preferred vegetable proteinaceous materials are soy protein and cottonseed protein, preferably those from a defatted source, more preferably defatted soybean protein. Representative of animal proteins are skim milk, casein and egg albumin. The proteinaceous material as obtained from a natural source may contain substantial amount of non-proteinaceous material and the terms "protein" and proteinaceous material" as used herein generally contemplate materials containing as little as about at least 25 % by weight of actual protein. Very suitable materials such as the preferred vegetable proteins usually contain between 40 % to 75 % actual protein.

While individual particles and the overall composition may have relatively low concentrations of the baculovirus, it is desirable from a minimum practical standpoint that the compositions contain the active equivalent of an $LD_{50}$ of at least 1.0 microgram/milliliter ($\gamma$/ml). The potency of the compositions may suitably range from 0.001 $\gamma$/ml up to 1.0 $\gamma$/ml, and is usually in the range of from 0.003 $\gamma$/ml to 0.4 $\gamma$/ml. The measurement of insecticidal activity or potency used and referred to is based on determination of the $LD_{50}$ value reported in micrograms per ml ($\gamma$/ml) of diet required to provide a level dose for 50 percent of the first instar larvae grown at a temperature of 30°C. The method is basically described in Insect Pathology; 6, 737-45 (1965) in connection with Trichopusia ni NPV potency estimation.

The particulate compositions provided by the invention exhibit a desired amount of insecticidal potency and are generally characterized by improved resistance to potency degradation by photo-inactivation and heat denaturation. Other properties of the compositions, particularly the physical properties, will vary depending upon various factors including particularly the materials employed for the matrix. Compositions in which the matrix is composed only of a clay are generally characterized by goods wettability but tend to have less stickerability and may, in some cases, be composed of softer particle. On the other hand, compositions in which the matrix is composed only of a vegetable protein may be generally characterized by good stickerability and hardness but tend to be less wettable. Compositions containing large amounts of animal protein tend to have good stickerability but poor wettability and hardness. Accordingly, the particularly preferred matrix materials are selected from the group consisting of vegetable protein, clay and mixtures thereof. It has also been found that compositions in which the matrix is composed of an intimate mixture of both a vegetable protein and clay have the advantage of offering good wettability, stickerability and hardness as well as other desirable properties. Accordingly, the especially preferred compositions of the invention are those in which the matrix is composed of a mixture of both a vegetable protein and clay. The ratio by weight of vegetable protein to clay may range from 0.1 to 10 parts of protein per parts by weight of clay and is preferably in the range of from 0.3 to 4 parts of vegetable protein per part of clay, more preferably 0.5 to 3 parts by weight of clay.

**FORMULATION EXAMPLE**

80 grams of a baculovirus preparation carrying the DF DNA sequence are blended into 500 ml water with a Waring lender for one minute. 1500 g Olancha clay and 820 g K-Soy$^R$ (defatted soybean powder) are suspended in 11 litres of water and then mixed with the baculovirus suspension. The final volume of the mixture is brought to 15 liters with water and each is agitated until a homogeneous suspension is obtained. The mixture is spray dried at an inlet temperature of 320° F. and an outlet temperature of 160° F while maintaining a pressure between 2000 to 4000 psi and using an orifice No. SA. 0.0444 to yield a spray-dried particle composition.

The construction of other plasmids and vectors incorporating sequences from Autographa californica, including the polyhedrin promoter thereof, and useful for similar cotransfection of Autographa californica DNA to form another hybrid insecticidal virus is also demonstrated in the examples, for purposes of illustration only. The following examples are offered by way of illustration, and are not intended to limit the invention.

Example A

Bioassays

M. sexta are reared on an artificial diet according to Bell et al. 1976. Ann. Entomol. Soc. Am. v69: 365-373. The cabbageworm Pieris rapae is reared on the diet described by Troetschler et al. (1985) J. Econ. Entomol. v78: 1521-1523 which is modified by addtion of fresh cabbage leaves as a phago-stimulant. Leaves of one immature cabbage plant are heated (15 min., 163° C) before grinding in a blender with each 3 liter batch of diet.

In vivo Bioassay in M. sexta. Post feeding late fifth stadium larvae of M. sexta lose 30% of their body weight during a 48 hour period. This apparent sensitivity to DF is used to assay for activity of synthetic DF. Prewandering, post feeding M. sexta are used just after lights out (12-12 photoperiod). These larvae are weighed, then injected into the insect blood system in the abdominal region with 24 μl "Manduca saline" (4mM NaCl, 40 mM KCl, 3mM CaCly, 18mM MgCl$_2$) or 25 ul saline with 0.1 ug synthetic DH. After 4 hours, these animals are again weighed and dry weight of frass was subtracted to determine total weight of water lost.

Example B

One assay for detection/evaluation of the insect control (insecticidal) activity is based on contact and penetration of the polypeptide. DMSO is employed as a carrier to facilitate such uptake. Accordingly, the factor to be evaluated is dissolved in about 30% aqueous DMSO and applied at various concentrations (1 mg/ml and ten-fold serial dilutions thereof) to a test group of twenty age-synchronized lepidopteran insects (Manduca sexta) at about the beginning of the third instar at about 27° C. The insects are evaluated for retarded growth and survival over the remaining molting stages and the results compared with a control group treated only with the DMSO solution.

Example 1

Extraction and Preliminary Purification

Pharate adult M. sexta are beheaded 24-48 hours before adult eclosion, and the heads frozen. A posterior section of the frozen heads containing the brain and the corpora cardiaca/corpora allata complex is punched out with a 5 mm diameter cork borer and stored at -80°C until extraction.

Ten thousand trimmed heads of M. sexta (fresh weight approximately 420 g) are homogenized in 1500 ml cold acetone and filtered. Residues are extracted with 1500 ml of 1 MHOAc/20 mM HCl (containing 0.1 mM phenylmethylsulfonyl fluoride and 0.01 mM pepstatin A, prepared freshly) and centrifuged at 10,000 x g for 20 min. After re-extraction of the pellet with 1200 ml of the same solution, and recentrifugation, the combined supernatants are applied to a SP-Sephadex C-25 column (25 x 700 mm, 300 ml bed volume) equilibrated with 1 M HOAc. The column is eluted with 1000 ml each of 1uM HOAc, 0.05 M $NH_4OAc$ (pH 4.0), and 0.05 M, 0.1 M, 0.4 M, and 0.8 M $NH_4OAc$ (pH 7.0). The 0.4 M and 0.8 M $NH_4OAc$ fractions, which both have DH activity, are applied directly to 10 g of reversed-phase Vydac $C_4$ packing material (20-30 um, contained in a 75 ml polypropylene syringe barrel with polyethylene frit) equilibrated with 0.1% trifluoroacetic acid (TFA). The cartridge is eluted with 100 ml each of 0.1% TFA and 20%, 35%, and 50% $CH_3CN$ in 0.1% TFA. DH is recovered in the 35% $CH_3CN$/0.1% TFA fraction.

## Example 2

### Purification

Vydac $C_4$ Semipreparative Liquid Chromatography (LC). This and subsequent purifications by LC are performed with a Perkin-Elmer Model 410 Bio pump, a Rheodyne loop injector, and a Kratos Model 783 variable wavelength detector, usually set at 220 nm. In an early attempt to isolate DF, biological activity was observed to diminish or be lost when solvent was evaporated from either the reversed-phase cartridge step or the semipreparative purifications, possibly due to association with other proteins, adsorption or oxidation. This problem was solved by modifying the pumping system with a Rheodyne Model 5302 valve installed in the "D" solvent line before the solvent proportioning valve, so that water-diluted fractions can be pumped into the column. The active fraction from Example 1 is diluted with 200 ml water and pumped onto a Vydac $C_4$ semipreparative column (10 x 250 mm) previously equilibrated with 20% $CH_3CN$ in 0.1% TFA. The column is eluted with an 80 min linear gradient of 20-40% $CH_3CN$ in 0.1% TFA at a flow rate of 5 ml/min; 10 ml fractions are collected. DF activity is found in fractions which elute from 34-38 min. These are combined and diluted with 40 ml water, and again pumped onto the same column, but now equilibrated with 10% 1-propanol (1-PrOH)/0.1% TFA. The retained materials are eluted with an 80 min linear gradient of 10-30% 1-PrOH in 0.1% TFA at a flow rate of 5 ml/min, with 10 ml fractions collected. DF is recovered in the 48-52 min fractions.

TSK SP-5PW LC. After addition of 2 ml of phosphate 0.1M buffer (pH 6.25), the active fraction from above is applied to a TSK SP-5PW ion exchange column (7.5 x 75 mm) equilibrated with 0.02 M phosphate buffer (pH 6.25) containing 10% $CH_3CN$. The column is eluted with two successive gradients at a flow rate of 1 ml/min; 10 min of 0-0.1 M NaCl in 0.02 M phosphate buffer (pH 6.25) containing 10% $Ch_3CN$, and 60 min of 0.1-0.4 M NaCl in the same buffer. Two ml fractions are collected. DF is recovered in the 48-52 fractions.

Vydac $C_4$ Analytical LC. The active fractions from above containing buffer salts and 10% $CH_3CN$, are pooled and applied via a 5 ml loop injector to a Vydac $C_4$ column (4.6 x 150 mm) equilibrated with 30% $CH_3CN$ in 0.1% heptafluorobutyric acid (HFBA). The column is eluted with a 75 min linear gradient of 30-45% $CH_3CN$ in 0.1% HFBA at a flow rate of 1.5 ml/min, with 3 ml fractions collected. DF is recovered only in the 36-38 min fraction.

Vydac $C_4$ Microbore LC. The active fraction from the previous step is diluted with 6 ml of water and applied to a Vydac $C_4$ microbore column (2.1 x 250 mm) using two injections with a 5 ml loop injector. The column is equilibrated with 20% $CH_3CN$ in 0.1% TFA. After elution with a 100 min linear gradient of 20-40% $CH_3CN$/0.1% TFA at a flow rate of 0.3 ml/min, pure DF is recovered in a peak at 69.0-71.5 min.

## Example 3

### Trypsin Digestion

Purified DF ( 1.5 nmol) from Example 2 is dissolved in 50 ul of 0.1 M Tris-HCl (pH 8.0)/0.01 M $CaCl_2$ containing 1 ug trypsin (TPCK treated, Sigma). After incubation at 35°C for 2 hours, the reaction mixture is applied to a Vydac $C_{18}$ column (4.6 x 100 mm). The fragment peptides are eluted with an 80 min gradient of 0-40% $CH_3CN$ in 0.1% TFA at a flow rate of 0.5 ml/min. The results are given below.

Table 2 - Amino Acid Composition of DF and Its Tryptic Peptides

| | Intact DF | Tryptic Peptides | | | | | |
|---|---|---|---|---|---|---|---|
| | | T - 1 | T - 2 | T - 3 | T - 4 | T - 5 | T - 6 |
| Asx | 4.92 (5) | 0.73 (1) | | | | 3.14 (3) | 0.79 (1) |
| Glx | 2.63 (3) | | | | 0.71 (1) | | |
| Ser | 3.69 (4) | | | | 1.05 (1) | | 3.14 (3) |
| Gly | 0.24 (0) | | | | | | |
| His | 0.63 (1) | | 0.38 (1) | 1.03 (1) | | | |
| Thr | 0.41 (0) | | | | | | |
| Ala | 4.00 (4) | 3.00 (3) | 1.09 (1) | 1.00 (1) | | | |
| Arg | 5.43 (5) | 1.20 (1) | 1.04 (1) | 1.09 (1) | | | 1.14 (1) |
| Pro | 2.00 (2) | | | | | | 2.26 (2) |
| Tyr | 0.05 (0) | | | | | | |
| Val | 1.52 (2) | | 0.57 (1) | 0.77 (1) | | | 0.96 (1) |
| Met | 1.56 (2) | | | | | | 1.59 (2) |
| Ile | 1.93 (2) | | | | | 0.97 (1) | 1.00 (1) |
| Leu | 6.72 (7) | | 1.00 (1) | 1.04 (1) | 2.00 (2) | 1.06 (1) | 3.00 (3) |
| Phe | 1.03 (1) | | | | | 0.99 (1) | |
| Lys | 3.38 (3) | | | 0.89 (1) | 0.95 (1) | | |
| Position | 1-41 | 31-35 | 26-30 | 25-30 | 18-22 | 36-41 | 2-15 |

Numbers in parentheses are from sequence analyses. Trp and Cys were not determined.

EP 0 359 714 B1

## Example 4

Sequence Analysis

Purified DF and tryptic peptides from Examples 2 and 3 are sequenced using an Applied Biosystems Model 477A pulsed liquid-phase protein sequencer. Released phenylthiohydantoin (PTH) amino acids are analyzed using an on-line analyzer (Applied Biosystems, Model 120A).

## Example 5

Amino Acid Analyses

Purified DF and tryptic peptides are hydrolyzed in vapor from 6 M HCl/1% phenol ($110\,^{\circ}$C for 20 hr). Hydrolysates are analyzed after conversion to phenylthiocarbamoyl amino acid derivatives by reversed-phase LC using an Ultrasphere ODS column (4.6 x 150 mm).

The starting buffer (D) is 0.05 M sodium acetate containing 1.15 mM triethylamine and 2% methanol, adjusted to pH 6 with phosphoric acid. The eluting buffers are identical except for the organic solvent; B contains 50% methanol and C contains 50% $CH_3CN$. The column washing solvent (A) is 70% $CH_3CN$ in $H_2O$. Buffers are delivered by a Perkin-Elmer series 4 pump using the following program. (Linear gradients were used between times indicated, except times marked with asterisk in Table 3, above. These are step gradients or isocratic segments).

### TABLE 3

| Time | Flow | A | B | C | D |
|---|---|---|---|---|---|
| 0 min | 1.0 ml/min | 0 | 0 | 0 | 100% |
| 21 min | 1.0 ml/min | 0 | 30% | 0 | 70% |
| 30 min | 1.0 ml/min | 0 | 0 | 35% | 65% |
| 45 min | 1.0 ml/min | 0 | 0 | 49% | 51% |
| 55 min* | 1.5 ml/min | 100% | 0 | 0 | 0 |
| 71 min* | 1.0 ml/min | 0 | 0 | 0 | 100% |
| 79 min* | 1.0 ml/min | 0 | 0 | 0 | 100% |

DF activity in fractions from each purification step of Example 2 is detected using an assay with newly emerged adult P. rapae in vivo, modified from an assay previously reported for Danaus plexippus by Dores et al. (1979) J. Insect Physiol. 25: 895-901 which is incorporated by reference. In addition to the application of a photoperiod-based heat pulse described by Dores et al., adult eclosion is further synchronized by placing pharate adults in a cardboard container seated on a warm water bath ($\leq 45\,^{\circ}$C). Newly eclosing adult butterflies are anesthetized with $CO_2$ within 1 min of the molt. They are then neck ligated with 15 min, immediately beheaded, and the wound sealed with melted wax. Each animal is suspended from the side of a 1 oz. plastic cup until the wings expand. All beheaded butterflies are weighed within 1 hour of the molt. Non-beheaded controls are weighed approximately 15 min after eclosion, but not otherwise treated.

Bovine serum albumin (BSA) (50 $\mu$g) is added to all test fractions before evaporating the solvent to reduce adsorption to containers. Treated animals are injected in the ventral thorax with 5 $\mu$l of the test fraction dissolved in 5 $\mu$l of "Manduca saline"; beheaded controls receive 5 $\mu$l saline. A lid is placed on the plastic cups to reduce evaporation. The butterflies are weighed again three hours after injection. The weight loss during this period is considered to be fluid excretion through the gut.

To determine net normal diuresis, the mean weight loss of treated controls is subtracted from the mean weight loss of untreated controls. The mean weight loss of a typical 114 mg untreated intact control animal in a three hour assay period is 23 mg (20% of mean body weight); that of a typical 118 mg beheaded control animal injected with 5 $\mu$l saline is 11 mg (9% mean body weight; and that of a typical 118 mg beheaded animal injected with 1 head-equivalent DF in 5 $\mu$l saline is 24 mg (20% mean body weight). The weight loss of treated, beheaded animals is expressed as a fraction of net normal diuresis.

Example 6

Biotechnicological Methods: except as may be otherwise indicated, the following general methods and procedures are employed in carrying out the biotechnicological experiments described herein with reference to construction of vectors, e.g., pDHC.

Gene Constructions: DNA fragments were chemically synthesized as were the complementary strands. The strands were annealed to form fragments of the genes and these were ligated into plasmids one at a time to result in the complete gene construct. The intermediate and final plasmid constructs were analyzed by restriction endonuclease digestion and gel electrophoresis for the presence and positions of diagnostic restriction sites. The constructs were also sequenced to verify the final sequence.

DNA Synthesis: Single stranded DNA fragments (oligonucleotides) were synthesized by automated solid phase methods using standard o-methyl- or $\beta$-cyanoethyl-deoxynucleoside phosphoramidite chemistry (Science 230:281-285, 1985). Either a Beckman System 1 Plus DNA Synthesizer or an Applied Biosystems Model 380A DNA Synthesizer was used for oligonucleotide synthesis using the chemical kits supplied by the respective manufacturers.

Gene Fragment Construction: Double stranded DNA gene fragments were constructed by mixing approximately 5 $\mu$g each of two complementary oligonucleotides in 400 $\mu$l of buffered NaCl solution (typically 10 mM Tris-HCl, pH 7.5, 20 mM NaCl, 0.1 mM EDTA; in a 1.5 ml Eppendorf microfuge tube. The mixtures were heated to 65 to 100°C for 5 to 20 minutes and slowly cooled to room temperature over a 10 to 60 minute period.

Ligations: Ligations of DNA fragments were performed by standard methods (Maniatis et al. 1982 Molecular Cloning, p. 146) or with the Ligation Kit manufactured by Takara Corporation. Typically, a 50 $\mu$l ligation reaction contained 40 pmol of dephosphorylated cohesive ended vector DNA, 20 pmol of cohesive ended insert DNA, and 0.025U T4 DNA Ligase in 25 mM Tris-Cl, pH 7.8, 10 mM MgCl$_2$, 4 mM $\beta$-mercaptoethanol, 0.4 mM ATP. In cases when a vector was not dephosphorylated, a similar reaction was set up except that it contained 80 pmol of the insert DNA. When using the Takara Ligation Kit, the amounts of vector and insert DNA similar to those just described were added to 20 $\mu$l of the Takara Kit Solution A and 5 $\mu$l of the Takara Kit Solution B. Standard ligation reactions were incubated at 4°C for 16 hours. The Takara Kit ligations were incubated at 16°C for 30 minutes. Exact ligation conditions varied depending on the specific reaction. In addition, in cases where blunt ended fragments were being ligated, conditions appropriate to blunt end ligations were used (e.g. 100 fold higher Ligase concentration). Each ligation was followed by a transformation reaction and analysis of the resulting transformants. In general, DNA from selected bacterial colonies isolated after the transformation reaction was prepared using the Mini Scale Preparation described below and was analyzed by restriction enzyme digestion and gel electrophoresis. Restriction enzymes appropriate to each specific ligation product were chosen to affirm that the resulting recombinant plasmids were of the intended configuration.

Transformations: Transformation competent JM101 bacterial cells were prepared by the standard CaCl$_2$ treatment method with the modification that glycerol was added to the cells to 15% and the cells were frozen and stored at -70°C prior to use. To transform cells with recombinant plasmid, between 5 and 50 $\mu$l of a ligation reaction was mixed with 50 $\mu$l of ice cold 50 mM CaCl$_2$. This solution was added to 200 $\mu$l of freshly thawed competent JM101 cells and the mixture was incubated in an ice bath for 30 minutes. The cells were heat shocked at 42°C for 60 to 90 seconds and then cooled on ice for 2 minutes. 1.2 mls of bacterial medium (TUM) were added and the mixture was incubated with shaking at 37°C for 1 hour. Then 100 to 200 $\mu$l of the culture was plated onto TA or TAXI plates and incubated overnight at 37°C.

Plasmid Mini Scale DNA Preparations: Colonies from transformation plates were picked and innoculated into 1 ml aliquots of TUM medium plus 50 $\mu$g/ml ampicillin in 1.5 ml Eppendorf microfuge tubes. The mini cultures were incubated at 37°C for 6 to 18 hours and small scale samples of plasmid DNA were prepared by the rapid boiling method (Maniatis et al., Supra. p. 366), but any of the known available methods may also be used.

Bacterial Medium:

| TUM Medium: | 10 g/l Bacto tryptone<br>5 g/l Bacto yeast extract<br>5 g/l NaCl<br>0.7 g/l KCl<br>2.5 g/l MgSO$_4$.7H$_2$O |
|---|---|
| TUM Plates: | TUM Medium plus<br>15 g/l Bacto agar |
| TA (TUM/Ampicillin) Plates: | TUM Plates plus<br>100 mg/l Ampicillin |
| TAXI Plates: | TA Plates plus<br>50 mg/l Xgal (5-bromo-4-chloro-3-indolyl-$\beta$-D-galactoside)<br>40 mg/l IPTG (isopropylthio-$\beta$-galactoside) |

Restriction Enzyme Digestions: Restriction endonucleases were purchased from New England Biolabs, International Biotechnologies, Inc., or Promega Biotec and used according to the supplier's specifications. Typically, 20 to 50 $\mu$l restriction enzyme reactions were set up using from 1 to 5 $\mu$g of plasmid DNA and from 1 to 20U of enzyme. The reactions also contained 1/10th volume of 10X reaction buffer, either the buffer from the enzyme supplier or one of the three standard restriction enzymes buffers (Maniates et al., Supra, p. 453).

Agarose Gel Electrophoresis: DNA fragments were analyzed on gels containing from 0.5 to 4% agarose (BRL Agarose, BRL LMP Agarose or FMC NuSieve GTG Agarose), 0.5 $\mu$g/ml ethidium bromide, 40 mM Tris-Acetate, pH 8.3, 1 mM ethylenediaminetetraacetic acid. The electorphoresis was conducted by standard methods as described by Maniatis et al., Supra, in horizontal electrophoresis tanks. The ethidium bromide stained DNA fragments were visualized using shortwave UV light and photographed on a UV transilluminator with a Polaroid 4X5 camera and Polaroid Type 57 film. Unknown fragment sizes were determined by comparison with adjacent DNA size standards (BRL 1kb Ladder or a Lambda phage HindIII digest). When fragments were purified using gel electrophoresis, the desired fragment was excised from the agarose gel and electroeluted into a small piece of dialysis tubing.

Conversion of 5' Overhangs to Blunt Ends: DNA fragments with 5' overhanging cohesive ends were converted to blunt ended fragments by a filling reaction using the Klenow fragment of E. coli DNA Polymerase I.

Conversion of 3' Overhands to Blunt Ends: DNA fragments with 3' overhanging cohesive ends were converted to blunt ended fragments by a 3'→5' exonuclease reaction using T4 DNA Polymerase or the Klenow fragment of E. coli DNA Polymerase I.

DNA Sequencing: Plasmids containing parts of the synthetic gene were sequence using the Sanger dideoxy method or a variation thereof. Plasmid infected cells were co-infected with the helper phage, M13K07, and single stranded DNA was produced and purified as indicated by the manufacturer, Pharmacia Inc. DNA sequencing reactions were performed using either the Sequenase Kit from US Biochemicals or the Dideoxy Sequencing Kit from Dupont/NEN. Reactions and gels were set up essentially as described in protocols supplied by the kit manufacturers.

Example 7

Production of Recombinant BmNPV virus: specific methods and procedures

I. Construction of Recombinant BmNPV.

A. Purification of Viral Particles for Extraction of DNA

Viral DNA for construction of a recombinant virus is prepared from either infected cell culture fluid or infected larvae.

Preparation of virus from cultured cells is as follows. Confluent cell monolayers in 150 mm dishes (2 x 10$^8$ cells in 30 ml medium) are prepared for infection. The medium is removed by aspiration and 500 $\mu$l to 1 ml of diluted viral solution containing about 10$^8$ PFU of wild type BmNPV is added into each of the 150 mm dishes. After incubation for 60 min with rocking at 15 min intervals, 17 ml of the medium is added. The cell fluids are collected at 60-72 hours post-infection and centrifuged at 3000 rpm for 20 min at 5°C. After an additional centrifugation at 3000 rpm for 20 min, the low speed supernatants are centrifuged at 100,000 g for 60 min. The pellets are suspended in about 2 ml of 10 mM Tris-HCl, pH 7.5, 1mM EDTA by strong pipeting and vortexing. Routinely 6 ml of the viral suspension is loaded onto a 20-50% (w/w) sucrose linear gradient prepared in 40 ml tube for a SW28 rotor. After centrifugation at 20,000 rpm for 90 min at 15°C, a viral band should be visible about 2/3 of the distance down the gradient. One to two ml of the gradient are collected with a syringe and needle by puncturing the side of the tube. The viral solution is diluted more than 20 fold with distilled water and precipitated by centrifugation at 100,000 g for 60 min. The supernatant is discarded and the solution remaining on the surface of the tube is removed with a Kimwipe. The precipitates are suspended in a small amount of 10 mM Tris-Hcl, pH 7.5, 1 mM EDTA and 200 $\mu$l each of the solution (0.3-0.5 mg DNA / ml) is transferred into a 1.5 ml polypropylene test tube (can be stored at -80°C). Average yield is about 5 $\mu$g of viral DNA from culture fluid of one 150 mm dish.

For viral DNA purification from larvae, the polyhedral inclusion bodies are first purified. A large quantity of the viral particles are purifed easily as follows. Larvae of the silkworm at the first day of the fifth instar are used. About 20 $\mu$l of viral solution containing $10^5$ to $10^6$ PFU and usually an appropriate amount of an antibiotic (1/20 volume of 60 mg/ml of Kanamycin) is injected into hemolymph using a syringe. After 4 to 5 days post-infection, the larvae are collected in a bottle filled with distilled water and stored at 5°C for 1 to 10 days. Polyhedra are purified from larvae by low speed centrifugation and can be stored at 5°C. About $10^{11}$ polyhedra (several hundred $\mu$l as a packed volume) are precipitated and are suspended in about 4 ml of 0.1 M $Na_2CO_3$, 0.05 M NaCl. After incubation for 30 min on ice, the solution will turn a clear opal color. It is then centrifuged at 3,000 rpm for 5 min and the supernatant is layered on a 20-50 % sucrose gradient as described above. After centrifugation, virus particles from the gradient are purified by the same method as for those from culture fluid.

B. Preparation of Viral DNA for Cotransfection

Viral DNA for cotransfection is prepared from viral particles purified as described above. Proteins are tightly bound to DNA of NPV, thus the following treatment is important for extraction of DNA. Furthermore, considering the large size of BmNVP viral DNA (about 130 kb), DNA should not be vortexed nor ethanol precipitated throughout extraction. Two hundred $\mu$l of the viral solution containing at least 0.3 mg DNA per ml, is usually used for extraction of DNA. Then 5.0 $\mu$l of 10% SDS and 20 $\mu$l of 20 mg/ml of Protease K (Merk) are added to the DNA in an Eppendorf tube. Mixed gently by flicking with a finger. After incubation at 37°C for 2 to 5 hours, the solution is extracted with an equal volume of phenol two to three times, extracted with an equal volume of phenol: chloroform (1:1), and extracted with an equal volume of chloroform twice. Each extraction is done by inverting the tube very slowly. Except for the final extraction with chloroform, the organic bottom layer is removed with a pipetman. The purifed DNA solution, which contains about 1% SDS, can be stored at least 4 months at 5°C. Digestion with restriction enzymes are diluted 1:5 to avoid any interference from the SDS.

C. Cotransfection of Recombinant Plasmid pBrnDH-1 and Wild Type Viral DNA

Recombinant viruses are constructed by homologous recombination between recombinant plasmid DNA and wild type viral DNA in calcium-mediated cotransfected cells. The method for cotransfection is similar to that used in mammalian cells.

Two different solutions are prepared in Eppendorf tubes. Solution A (250 $\mu$l) is composed of 2 $\mu$g of viral DNA, 5 $\mu$g of recombinant plasmid pBmDH-1, 0.25 M $CaCl_2$, obtained when 5 $\mu$l of viral DNA, 5 $\mu$l of the recombinant plasmid DNA, 30 $\mu$l of 2M $CaCl_2$, and 210 $\mu$l of distilled water are mixed. Solution B (250 $\mu$l) is composed of 50 mM Hepes buffer, pH 7.1, 0.28M NaCl, 0.7 mM $Na_2PO_4$, and 0.7 mM $NaHPO_4$, obtained when 250 $\mu$l of 50 mM Hepes, 0.28 M NaCl in a tube is added 5 $\mu$l each of 35 mM $Na_2PO_4$ and 35 mM $NaHPO_4$. To make the calcium precipitate, solution A is added dropwise with a pipetman into the solution B which is gently bubbling with air from a Pasteur pipet. The color of the solution changes to slight opal upon precipitation. After incubation for 30 min at room temperature, 500 $\mu$l of the mixture is added into the culture of BmN cells in a 60 mm dish. Stock solutions of antibiotics (for example, 10 $\mu$l of 60 mg/ml Kanamycin) are added to prevent contamination problems. Six to 16 hours after adding the cotransfection mixture, the medium is replaced with fresh medium and the cells are cultured for 5 more days. Polyhedral inclusion bodies can be seen 4 to 5 days after transfection. The cell culture fluid is subjected to plaque assay for isolation of a recombinant virus.

### D. Isolation of a Recombinant Virus Carrying DH Gene

Recombinant virus in which the polyhedrin gene is replaced with the DH gene is detected by the absence of production of inclusion bodies in infected nuclei using a light microscope. To isolate the recombinant virus from the wild type virus, the plaque assay described herein is used. The cell monolayer infected with a cotransfected virus sample is overlaid by 0.75% SeaPlaque agarose and cultured for 4 to 6 days. Generally, only between 0.2 to 3% of the produced plaques are occlusion negative.

The culture fluid at 5 to 6 days after cotransfection usually contains about $10^7$ PFU per ml. The fluid is collected and diluted for plaque assay. Routinely, the following three sets of solutions are prepared by dilution with TC-100 containing 1 % FCS. (A) $10^{-3}$ dilution; 10 $\mu$l of the transfected cell fluid is mixed into 1 ml of medium; (B) 10-4 dilution; 100 $\mu$l of solution (A) is added to 900 $\mu$l of medium; (C) 10-5 dilution; 100 $\mu$l of solution (B) is added to 900 $\mu$l of medium. One hundred (or 200) $\mu$l of each solution is added to BmN cells in a 60 mm dish for plaque assay.

Plaques produced by the recombinant virus are screened by a light microscope using a combination of low (x400) and high (x1200) magnification. Staining with neutral red on the second overlay is omitted to ease the identification of recombinant viruses by light microscopy. Five days after overlay, plaques can be easily identified without a light microscope. When dishes are observed at low magnification, the wild type plaques show dark color. Recombinant plaques have a slightly lighter color. It is sometimes easier to find recombinant plaques by screening with the microscope slightly out of focus. The candidates are confirmed at high magnification by the following characteristics. Polyhedral inclusion bodies are not observed, but some cells produce crystals similar to polyhedral inclusion bodies (but without clear outlines). Areas which are not completely covered with cells are typically plaques because of the inhibition of cell growth. Sometimes cells with expanded nuclei, cells with rounded shape with a distinct cell membrance, or shrunken cells are observed in the plaque.

The plaques produced by a recombinant virus are marked with a felt tipped pen, and are picked with a Pasteur pipet. The Pasteur pipet is stuck vertically into the plaque, and agarose plug is forcefully pipeted in 1 ml of TC-100 containing 1% FCS to be broken down. This solution contains about $10^3$ PFU. A second and third plaque purification are to isolate a pure clone.

Isolation of a recombinant virus from the cotransfected is also done on a 96 well plate after infection with appropriate dilutions. Cells in a well showing cytopathic effect but not polyhedral production indicate recombinant viral infection and the pure isolate is cloned by an additional plating of a diluted sample from the recombinant well. Recombinant BmNPV virus with the DH gene at the expected site is recovered from the foregoing experiment as confirmed by hybridization and restriction endonuclease analysis.

### II. Expression of Recombinant BmNPV in Larvae of Silkworm.

A. The recombinant RmNPV (2 x 105 PFU) in about 5 $\mu$l of tissue culture medium is injected into the body cavity of silkworm larvae on the first day of the fifth instar. Controls using the native virus are also carried out. Results are evaluated daily over a six day period and indicate that the recombinant virus increases the number of dead larvae observed prior to the sixth day. The results of duplicate experiments showing such results are summarized below in Table 4.

## TABLE 4

Experiment One:

Injected 2 X 105 PFU of virus in 5ul tissue culture medium per first day fifth instar Bombyx mori larvae. Incubated at 27°C.

NUMBER OF DEAD LARVAE (5 per sample)

| VIRUS | DAYS AFTER INJECTION | | | | |
|---|---|---|---|---|---|
| | 2 | 3 | 4 | 5 | 6 |
| Control (no virus) | 0 | 0 | 0 | 0 | 0 |
| BmT3 Cntrol (wild type virus) | 1 | 2 | 2 | 2 | 5 |
| IFN Control (interferon recombinant) | 0 | 0 | 1 | 5 | 5 |
| BmNPV (Diuretic Factor NPV) | 0 | 0 | 1 | 5 | 5 |

Experiment Two:

Injected 2X 105 PFU of virus in 5ul tissue culture medium per first day fifth instar Bombyx mori larvae. Incubated at 27°C.

NUMBER OF DEAD LARVAE (6 per sample)

| VIRUS | DAYS AFTER INJECTION | | | | |
|---|---|---|---|---|---|
| | 2 | 3 | 4 | 5 | 6 |
| BmT3 Control (wild type virus) | 0 | 0 | 0 | 6 | 6 |
| INF Control (interferon recombinant) | 0 | 0 | 0 | 6 | 6 |
| BmNPV (Diuretic Factor NPV) | 0 | 0 | 6 | 6 | 6 |

III. Insect Cell Line

A. Bombyx mori cells from the BmN-4 cell line are employed and maintained in a culture medium TC-100 which is supplemental with 10% fetal calf serum (FCS). The composition of the culture medium TC-100 is as follows:

| | Component | Amount/4 liter | Amount/1 liter |
|---|---|---|---|
| (A) | KC1 | 11.48 g | 2.87 g |
| | $CaCl_2.2H_2O$ | 5.28 g | 1.32 g |
| | $MgCl_2.6H_2O$ | 9.12 g | 2.28 g |
| | $MgSO_4.7H_2O$ | 11.12 g | 2.78 g |
| | Glucose | 4.0 g | 1.0 g |
| | Trytose broth | 10.04 g | 2.51 g |
| | L-Alanine | 0.9 g | 0.23 g |
| | $\beta$-Alanine | 0.8 g | 0.2 g |
| | L-Arginine HCl | 2.8 g | 0.7 g |
| | L-Aspartic acid | 1.4 g | 0.35 g |
| | L-Asparagine | 1.6 g | 0.4 g |
| | L-Glutamic acid | 2.4 g | 0.6 g |
| | L-Glutamine | 2.4 g | 0.6 g |
| | Glycine | 2.6 g | 0.65 g |
| | L-Histidine | 10.0 g | 2.5 g |
| | L-Isoleucine | 0.2 g | 50 mg |
| | L-Leucine | 0.3 g | 75 mg |
| | L-Lysine HCl | 2.5 g | 0.625 g |
| | L-Methionine | 0.2 g | 50 mg |
| | L-Phenylalanine | 0.6 g | 1.15 g |
| | L-Proline | 1.4 g | 0.35 g |
| | DL-Serine | 4.4 g | 1.1 g |
| | L-Threonine | 0.7 g | 0.175 g |
| | L-Valine | 0.4 g | 0.1 g |
| (B) | L-Cystine | 0.1 g | 25 mg |
| | L-Tryptophan | 0.4 g | 0.1 g |
| | L-Tyrosine | 0.2 g | 50 mg |
| (C) | $NaH_2PO_4.2H_2O$ | 4.55 g | 1.14 g |
| (D) | $NaHCO_3$ | 1.4 g | 0.35 g |
| (E) | 1000 x Vitamines | 4 ml | 1 ml |

in which the component E (1000 x vitamines is composed as follows:

| Component | Amount/100 ml |
|---|---|
| Thiamine HCl | 2.0 mg |
| Riboflavin | 2.0 mg |
| Calcium pantothenate | 2.0 mg |
| Pyridoxine HCl | 2.0 mg |
| p-Aminobenzoic acid | 2.0 mg |
| Folic acid | 2.0 mg |
| Nicotinic acid | 2.0 mg |
| Inositol | 2.0 mg |
| Biotin | 1.0 mg |
| Choline HCl | 20.0 mg |

The subculture temperature is about 27°C. Confluent cells are suspended in fresh complete medium and seeded in fresh flasks at a dilution ratio of 1:2. The cells are generally passaged every 4 to 5 days.

BmN-4 cells can be stored for at least 6 months at -70°C or in liquid nitrogen without loss of viability. Cells harvested from confluent cultures are suspended in fresh medium containing 10% FCS on ice at a cell density of $2 \times 10^6$ per ml. The cell suspension is mixed with a 10% volume of DMSO and 1 ml aliquots are pipeted into 2 ml screwcapped plastic tubes. These tubes are wrapped with two to three layers of paper towels to control the rate of temperature decrease and are transferred to a deep freezer. For stock in liquid nitrogen, the tubes can be transferred after incubation for several hours at -70°C. To recover cells from the stock, the cells in the tube are quickly thawed in a cold water bath and then seeded into a 25 cm$^2$ flask with an additional 3 to 4 ml of fresh medium. After the cells are attached to the surface, the medium is replaced with fresh medium. If unattached dead cells are found in the medium, these cells can be removed by changing the medium without passage.

B. Preparation of TC-100 culture medium

TC-100 culture medium is made by the following procedure. As the medium is sterilized by filtration, viral contamination is avoided during the preparation of medium.

It is convenient to prepare a separate stock solution for concentrated (1000x) vitamins as follows. Each of the ten ingredients is dissolved in 100 ml of double distilled water. These stock solutions can be stored at -20°C. As precipitation is usually seen in the solution even after gentle mixing, mix well before use.

To make 4 liters of the complete medium, each of the chemicals in Component (A) is added into 3 liters of double distilled water with stirring. Insoluble amino acids in Component (B) are separately dissolved in 150 ml of 0.2N HCl at around 50°C. $NaH_2PO_4.H_2O$ and $NaHCO_3$ are dissolved in 100 ml each of distilled water. These separately prepared solutions and 4 ml of 1000 x vitamins stock are added into the above solution.

After complete dissolution of the chemicals, pH is adjusted to 6.2 with 5 N KOH (usually less than 2 ml). Finally, the volume is adjusted to 4 liters with distilled water. For plaque assay, 1.33 (4/3) x TC-100 is prepared simply by making 750 ml using the amount of chemicals for 1 liter (Table 2) of the complete medium. Higher concentration of TC-100 may cause precipitation during storage at 5°C, which will damage the cells.

The medium is sterilized by filtration (0.22 $\mu$m filter) and can be stored at 5°C for up to 4 months. Prefiltration is useful to avoid clogging of filters.

IV. Plaque Assay

The procedure of a plaque assay of BmNPV on BmN-4 cells is as follows. A monolayer of 2 x 10$^6$ cells in a 60 mm dish (4-5 ml for culture) is used for plaque assay. The cells are seeded at least 2 hours prior to plaque assay. The dish should be rocked after seeding to produce a uniform cell density. Confluent cells in a 60 mm dish can provide two 60 mm dishes for plaque assay.

For viral infection, the culture fluid is discarded (if many, aspiration is recommended) and 100 $\mu$l (or 200 $\mu$l) of the viral (usually diluted) solution is added. After incubation for 1 hour with rocking at 15 minute intervals, the monolayers are overlaid with TC-100 medium containing 0.75% SeaPlaque agarose and 5% FCS.

The overlay solution is prepared as follows. SeaPlaque agarose is suspended at 3% in double distilled water in a bottle (or a glass test tube) and autoclaved. The bottle is kept at 40-50°C after autoclaving. The amount of the agarose and distilled water is calculated by the following equations.

Agarose (mg) = (Number of plates + 1) x 34 mg
Water (ml) = (Number of plates + 1) x 1.13 ml

Pre-warmed (37°C) 1.33 x TC-100 medium containing 5% FCS and 60 $\mu$g/ml of Kanamycin is added into the agarose solution and mixed immediately. The amount is calculated as follows:

1.33 x TC-100 (ml) = (Number of plates + 1) x 3.44 ml
FCS (ml) = (Number of plates + 1) x 0.23 ml
1000 x Kanamycin (60 mg/ml) = (Number of plates + 1) x 9 $\mu$l

To prevent detachment of the cells, the agarose solution (4.5 ml) is pipeted slowly onto an infected monolayer. The overlaid plates are kept on a bench for 20 minutes with the lid slightly opened to solidify. Plaques can be identified after incubation for 4 to 6 days at 27°C. The titer of the original solution is calculated by dilution rate and the volume added to the plate. To help visualize the plaques more clearly, a second agarose overlay (2 ml) containing 0.02% of neutral red is recommended. The counting of plaques should be done at least 12 hours after the second overlay.

The viral solution added to the plate for titration should be diluted to give between 10 to 200 plaques per dish. The viral titer in infected medium can be estimated by evaluating the conditions of infection. Generally, the maximum titer is around 10$^8$ Plaque Forming Units (PFU) per ml in the medium. The lowest titer observed, for example in the medium of the initially infected cells after several washes, is $10^3$ to $10^4$ PFU/ml.

Viral titers can be also estimated as $TCID_{50}$ using a 96 well plate.

Purified agarose, SeaPlaque agarose (FMC corporation) is found to be suited as an overlay material because of a low toxicity to insect cells. The overlay solution contains TC-100, 5% FCS, and 0.75% SeaPlaque agarose.

Example 8

Preparation of pBE284

The preparation of plasmid pBE284 (see Figs. 1 and 3) will be evident from information contained herein and the following description. The starting plasmids are the known plasmids pBmE36 (see Figs. 1 and 2) and pUC19 (Pharmacia Inc.). Each is cut with Hind III and the 3.9 Kb fragment from pBmE36 ligated to the linearized pUC19 to form pE36-1. This plasmid is then cut with BamHI and Sma I and the resulting large fragment ligated with the 1.8 Kb fragment obtained on cutting λ (Lambda) phage with Bam HI and Sma I to form pE36-2 (the λ DNA serving as a spacer). The plasmid pE36-2 is then cut with Sna BI and the linearized DNA treated with Bal 31 exonuclease at 30°C, in 600 mM NaCl, 12 mM CaCl₂, 12 mM Mg Cl₂, 20 mM Tris-Cl, 1 mM EDTA, pH 8.0. This treatment was conducted as several different experiments monitored at different times to ascertain the reduced DNA lengths obtained (after cutting with Sma I to remove the spacer DNA, ligating to circularize, transforming bacteria and sequencing the isolates). One isolate, pE36-3, showed the position desired for the truncated DNA in pBE284 relative to the original position of the start codon (ATG) for the polyhedrin gene.

The plasmid pE36-3 was cut with EcoRI and Kpn I, the EcoRI overhang filled in and the Kpn I overhang blunted and the resulting DNA ligated to form pE36-4 (with its new EcoRI site and the Sac I and Kpn I sites of pE36-3 removed). The plasmid pE36-4 is cut with EcoRI and Sca I and the fragment of about 2 Kb isolated for later use.

The known plasmid pBm030 (described in published European Patent Application No. 0222412) is cut with EcoRI and Xba I, each of the overhangs filled in (Klenow) and the ends ligated to form p030-1 (with its new EcoRI and Xba I sites). The plasmid p030-1 is cut with EcoRI and Sca I and the resulting fragment of about 5 Kb isolated and ligated with the fragment of about 2 Kb as isolated above from pE36-4 to form the desired plasmid pBE284.

Example 9

Preparation of Plasmid pDH-1

The plasmid pBmDH-1 is prepared as follows: the plasmid pDHC is cut with Hind III, the overhand filled in with Klenow, the resulting DNA cut with EcoRI and the fragment containing the DH gene isolated. Such fragment is then ligated with the larger fragment obtained on cutting pBE284 with EcoRI and AatI, to form pBmDH-1. In pBmDH-1 the sequence resulting in the area of ligation and the deleted start codon of the polyhedrin gene and the introduce start codon of the diuretic factor gene is as follows:

```
      *          * * *
TAAGGAATTCATG
ATTCCTTAAGTAC
-------> Met
```

wherein the arrow is the direction of transcription, the single asterisk indicates the normal position or beginning of the deleted start codon of the polyhedrin gene and the triple asterisk indicates the start codon in the diuretic factor gene.

Example 10

Preparation of pAcE7 with Autographa californica promoter to A. californica systems

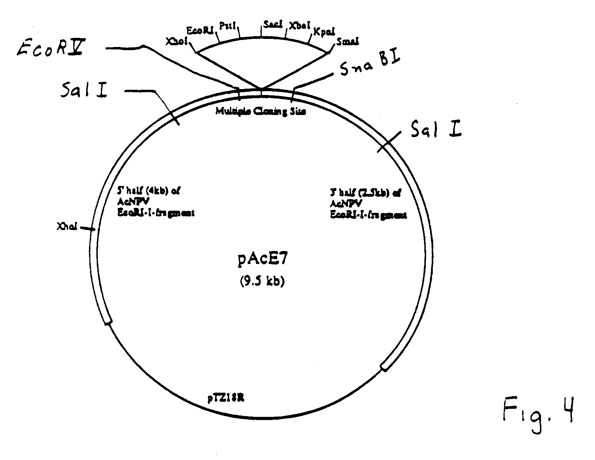

The plasmid pAcE7 (depicted in Fig. 4) was prepared as follows. A plasmid pAcX33 containing 7.2kb of the AcNPV genome was constructed as follows. The known plasmid pEXS942B6 containing a 12kb XhoI/EcoRI fragment from the AcNPV genome (including the polyhedrin gene) was digested with EcoRI and the 7.2kb EcoRI-I-fragment was purified by agarose gel electrophoresis. This fragment was cloned into EcoRI cleaved pTZ18R. Two series of reactions were then performed to remove the pTZ18R polylinker restriction sites. First, the plasmid was partially digested with EcoRI (the plasmid contains two sites) and linear plasmid was isolated. The EcoRI cohesive ends were filled in using Polymerase I klenow fragment and the blunt end molecules were recircularized. The resulting isolates were analyzed by EcoRI/HindIII digestion and a plasmid specifically missing the EcoRI site far from the polylinker was selected. Secondly, this new plasmid was digested with EcoRI and HindIII (th two outermost polylinker sites), blunt ended with Klenow, and re-circularized to yield a plasmid lacking all polylinker sites. This plasmid construct lacking all the pTZ18R polylinker sites and containing the 7.2kb EcoRI-I-fragment from AcNPV was denoted pAcX33.

A 3kb SalI fragment completely containing the polyhedrin gene was subcloned out of pEXS942B6 into the pTZ19R vector in two orientations to create the plasmids pP1 and pP2. A 42 nucleotide oligomer with 5 mismatched bases was synthesized and used for oligomer directed mutagenesis (Amersham Kit) to create a new XhoI site at position-5 relative to the polyhedrin start (ATG) codon. A 26 nucleotide oligomer with 2 mismatched bases was synthesized and used for oligomer directed mutagenesis to create a new XhoI site at a site 3 bases downstream of the polyhedrin stop (TAA) codon. The 42 and 26 nucleotide oligomers used had the following sequences nucleotide respectively:

5'TGAATAATCCGGCATTCTCGAGAGGTTTTTTTATTACAAAAC3' (42 mer), and

5'AATAACAATGTCTCGAGTTTTAATAC3' (26 mer)

The double mutagenized plasmid was cut at the two novel sites with XhoI and re-circularized to create a plasmid containing the polyhedrin 5' and 3' non-coding regions flanking a unique XhoI site. A synthetic double stranded 45bp oligomer encoding sites for XhoI, EcoRI, PstI, SacI, XbaI, KpnI, and SmaI followed by stop codons (TAA) in three reading frames was inserted into the XhoI site to create multiple cloning sites behing the polyhedrin promoter. The double stranded 45 bp oligomer used had the following sequence:

5'TCGAGAATTCTGCAGAGCTCTAGAGGTACCCGG

3' CTTAAGACGTCTCGAGATCTCCATGGGCC

GTAATTAATTAA3'

CATTAATTAATTAGCT5'

Finally, the fragment containing the polyhedrin 5' and 3' sequences and the multiple cloning site was excised with EcoRV and SnaBI and inserted into the plasmid pAcX33 (which had been cut with EcoRV and SnaBI, to obtain pAcE7.

The new Autographa californica NPV transfer vector pAcE7 contains 6 unique cloning sites (EcoRI, PstI, SacI, XbaI, KpnI, and SmaI) 3' to the polyhedrin promoter. It also contains stop codons in all reading frames 3' to the cloning sites. pAcE7 contains about 6.5kb of AcNPV DNA to promote homologous recombination with native AcNPV DNA. This is composed of 4kb of 5' sequences and 2.5kb of 3' sequences. The plasmid is illustrated in Fig. 4.

Example 11

Construction of Shuttle Vector pAcDH-1 for Cotransfection from Plasmids pAcE7 and pDHC

The plasmid pDHC was digested with the restriction endonuclease Hind III and the resulting linearized plasmid treated with Klenow to fill in the overhand. Such linear DNA was then digested with EcoRI and the fragment containing the diuretic factor structural gene isolated and ligated with the large fragment resulting from digesting the plasmid pAcE7 with EcoRI and Sma I to obtain the desired shuttle vector or plasmid pAcDH-1.

Example 12

Cotransfection and Production of Recombinant Hybrid Autographa californica NPV DNA from pAcDH-1 and viral DNA

Following the procedures described by Miller et al. in the section entitled "An Insect Baculovirus Host-Vector System for High-Level Expression of Foreign Genes", pages 277-298 in "Genetic Engineering: Principles and Methods, Vol. 8 (1986), Plenum Press N.Y. (Edited by J.K. Setlow and A. Hollaender), the plasmid pAcDH-1 and Autographa californica NPV DNA (prepared as described therein) are combined under for transfection (cotransfection) to obtain and isolate after plaque assay a viable recombinant hybrid virus DNA.

In comparative evaluations conducted analogously to those in Example 6, Section II, Part A, the recombinant hybrid AcNPV virus DNA is also indicated to provide enhanced insecticidal activity by reducing the time for lethal effect of the alfalfa looper caterpillar (A. californica) compared to native A. californica virus.

**Claims**

1. A polypeptide which has insect diuretic factor activity, substantially free from associated insect polypeptides, having an amidated C-terminus and an amino acid sequence according to Formula (I),

```
Arg-Met-Pro-Ser-Leu-Ser-Ile-Asp-Leu-Pro
Met-Ser-Val-Leu-Arg-Gln-Lys-Leu-Ser-Leu      (formula I)
Glu-Lys-Glu-Arg-Lys-Val-His-Ala-Leu-Arg
Ala-Ala-Ala-Asn-Arg-Asn-Phe-Leu-Asn-Asp
Ile-NH$_2$
```

or an analogue or derivative of the polypeptide of Formula (I), in which one or more amino acid residues has been deleted or added or substituted by a different amino acid residue, without substantially reducing the peptide's diuretic activity.

2. A precursor polypeptide which has insect diuretic factor activity, substantially free from associated insect polypeptides, having an amino acid sequence according to Formula (II),

```
Arg-Met-Pro-Ser-Leu-Ser-Ile-Asp-Leu-Pro
Met-Ser-Val-Leu-Arg-Gln-Lys-Leu-Ser-Leu
Glu-Lys-Glu-Arg-Lys-Val-His-Ala-Leu-Arg      (formula II)
Ala-Ala-Ala-Asn-Arg-Asn-Phe-Leu-Asn-Asp
Ile-X
```

wherein X is Gly, Gly-Lys, or Gly-Lys-Arg.
or an analogue or derivative of the polypeptide of Formula (II), in which one or more amino acid residues has been deleted or added or substituted by a different amino acid residue, without substantially reducing the peptide's diuretic activity.

3. A polypeptide having insect diuretic activity which is coded for by a) a DNA sequence comprising the 123 nucleotides extending from nucleotide position number 54 to nucleotide position number 177 in Table A; or b) a nucleotide sequence which hybridizes under stringent conditions to such 123 nucleotide sequence.

4. An isolated DNA comprising an intronless DNA sequence encoding a polypeptide comprising the 41 amino acid sequence extending from amino acid position number 17 to amino acid position number 57 in Table A.

5. A DNA in accordance with Claim 4 in which the intronless DNA sequence encodes a polypeptide comprising the 42 amino acid sequence extending from amino acid position number 17 to amino acid position number 58 in Table A.

6. A vector DNA comprising a DNA sequence encoding a polypeptide according to any one of Claims 1-3.

7. A vector DNA in accordance with Claim 6 in which said DNA sequence is under expression control of heterologous promoter DNA, wherein said DNA sequence comprises an intronless DNA sequence encoding the polypeptide comprising the 41 amino acid sequence extending from amino acid position number 17 to amino acid position number 57 in Table A.

8. A vector DNA in accordance with Claim 7 in which the intronless DNA sequence encodes a polypeptide comprising the 42 amino acid sequence extending from amino acid position number 17 to amino acid position number 58 in Table A.

9. A vector DNA in accordance with Claim 7 in which the promoter DNA is an NPV polyhedrin promoter.

10. A vector DNA in accordance with Claim 9 in which said DNA sequence encoding said polypeptide is 5' and 3' flanked by an insect virus DNA sequence comprising insect virus polyhedrin structural gene DNA.

11. A vector DNA in accordance with Claim 9 in which the promoter is a _Bombyx mori_ polyhedrin promoter or an _Autographa californica_ polyhedrin promoter.

12. A hybrid baculovirus DNA competent to lethally infect insect cells and comprising baculovirus DNA and a DNA sequence coding for a polypeptide according to any one of Claims 1-3, said coding sequence being under expression control of a promoter operable to effect expression of said coding sequence in the baculovirus.

13. A baculovirus DNA of Claim 12 in which said DNA polypeptide coding sequence comprises: a) the 123 nucleotides extending from nucleotide position number 54 to nucleotide position number 177 in Table A; or b) a nucleotide sequence which hybridizes under stringent hybridization conditions to such 123 nucleotide sequence.

14. A baculovirus DNA of Claim 12 in which said DNA polypeptide coding sequence comprises an intronless DNA sequence encoding the polypeptide comprising the 41 amino acid sequence extending from amino acid position number 17 to amino acid position number 57 in Table A.

15. A baculovirus of Claim 14, in which the intronless DNA sequence encodes a polypeptide comprising the 42 amino acid sequence extending from amino acid position number 17 to amino acid position number 58 in Table A.

16. A baculovirus of Claim 15, in which the intronless DNA sequence encodes a polypeptide comprising the 58 amino acids extending from amino acid position number 1 to amino acid position number 58 in Table A.

17. A baculovirus in accordance with Claim 14 which is _Bombyx mori_ NPV DNA or _Autographa californica_ NPV DNA.

**18.** An insecticidal composition comprising an insecticidally effective amount of a hybrid baculovirus of Claims 12-17 and an inert carrier.

**19.** A method of combating insects susceptible to a NPV baculovirus comprising applying to the locus of the insects an insecticidally effective amount of a hybrid baculovirus DNA of Claims 12-17 in an inert carrier.

**Patentansprüche**

**1.** Polypeptid, das die Wirkung eines diuretischen Faktors für Insekten aufweist und im wesentlichen frei von assoziierten Insektenpolypeptiden ist, einen amidierten C-Terminus und eine Aminosäuresequenz gemäß der Formel (I) aufweist

```
Arg-Met-Pro-Ser-Leu-Ser-Ile-Asp-Leu-Pro
Met-Ser-Val-Leu-Arg-Gln-Lys-Leu-Ser-Leu
Glu-Lys-Glu-Arg-Lys-Val-His-Ala-Leu-Arg    (Formel I)
Ala-Ala-Ala-Asn-Arg-Asn-Phe-Leu-Asn-Asp
Ile-NH₂
```

oder ein Analogon oder Derivat des Polypeptids der Formel (I), worin eine oder mehrere Aminosäurereste deletiert, hinzugefügt oder durch einen unterschiedlichen Aminosäurerest substituiert worden sind, ohne die diuretische Wirkung des Peptids wesentlich zu reduzieren.

**2.** Vorläuferpolypeptid, das die Wirkung eines diuretischen Faktors für Insekten aufweist und im wesentlichen frei von assoziierten Insektenpolypeptiden ist, das eine Aminosäuresequenz gemäß der Formel (II) aufweist

```
Arg-Met-Pro-Ser-Leu-Ser-Ile-Asp-Leu-Pro
Met-Ser-Val-Leu-Arg-Gln-Lys-Leu-Ser-Leu
Glu-Lys-Glu-Arg-Lys-Val-His-Ala-Leu-Arg    (Formel II)
Ala-Ala-Ala-Asn-Arg-Asn-Phe-Leu-Asn-Asp
Ile-X
```

worin X Gly, Gly-Lys oder Gly-Lys-Arg ist,
oder ein Analogon oder Derivat des Polypeptids der Formel (II), worin eine oder mehrere Aminosäurereste deletiert, hinzugefügt oder durch einen unterschiedlichen Aminosäurerest substituiert worden sind, ohne die diuretische Wirkung des Peptids wesentlich zu reduzieren.

**3.** Polypeptid mit diuretischer Wirkung für Insekten, das kodiert wird von a) einer DNA Sequenz, die die von der Nukleotidposition Nummer 54 bis zur Nukleotidposition Nummer 177 in Tabelle A reichenden 123 Nukleotide enthält, oder b) einer Nukleotidsequenz, die unter stringenten Bedingungen mit dieser 123 Nukleotide langen Sequenz hybridisiert.

**4.** Isolierte DNA, die eine intronfreie DNA Sequenz enthält, die ein Polypeptid kodiert, das die von der Aminosäureposition Nummer 17 bis zur Aminosäureposition Nummer 57 in Tabelle A reichende 41 Aminosäuren lange Sequenz enthält.

**5.** DNA nach Anspruch 4, worin die intronfreie DNA Sequenz ein Polypeptid kodiert, das die von der Aminosäureposition Nummer 17 bis zur Aminosäureposition Nummer 58 in Tabelle A reichende 42 Aminosäuren lange Sequenz enthält.

**6.** Vektor DNA, die eine ein Polypeptid nach einem der Ansprüche 1-3 kodierende DNA Sequenz enthält.

25

**7.** Vektor DNA nach Anspruch 6, worin diese DNA Sequenz unter der Expressionskontrolle einer heterologen Promotor DNA steht, worin die DNA Sequenz eine intronfreie DNA Sequenz enthält, die das Polypeptid kodiert, das die von der Aminosäureposition Nummer 17 bis zur Aminosäureposition Nummer 57 in Tabelle A reichende 41 Aminosäuren lange Sequenz enthält.

**8.** Vektor DNA nach Anspruch 7, worin die intronfreie DNA Sequenz ein Polypeptid kodiert, das die von der Aminosäureposition Nummer 17 bis zur Aminosäureposition Nummer 58 in Tabelle A reichende 42 Aminosäuren lange Sequenz enthält.

**9.** Vektor DNA nach Anspruch 7, worin die Promotor DNA ein NPV Polyhedrinpromotor ist.

**10.** Vektor DNA nach Anspruch 9, worin die dieses Polypeptid kodierende DNA Sequenz 5' und 3' von einer Insektenvirus DNA Sequenz flankiert ist, die die DNA Sequenz für das Polyhedrinstrukturgen der Insektenvirus DNA enthält.

**11.** Vektor DNA nach Anspruch 9, worin der Promotor ein Bombyx mori Polyhedrinpromotor oder ein Autographa californica Polyhedrinpromotor ist.

**12.** Hybrid-Baculovirus DNA, die zur letalen Infektion von Insektenzellen fähig ist, und Baculovirus DNA und eine DNA Sequenz enthält, die für ein Polypeptid nach einem der Ansprüche 1-3 kodiert, wobei die kodierende Sequenz unter der Expressionskontrolle eines Promotors steht, der zur Bewirkung der Expression dieser kodierenden Sequenz in Baculovirus fähig ist.

**13.** Baculovirus DNA nach Anspruch 12, worin die für das Polypeptid kodierende DNA Sequenz enthält a) die von der Nukleotidposition Nummer 54 bis zur Nukleotidposition Nummer 177 in Tabelle A reichenden 123 Nukleotide, oder b) eine DNA Sequenz, die unter stringenten Hybridisierungsbedingungen mit dieser 123 Nukleotide langen Sequenz hybridisiert.

**14.** Baculovirus DNA nach Anspruch 12, worin die für das Polypeptid kodierende DNA Sequenz eine intronfreie DNA Sequenz enthält, die das Polypeptid kodiert, das die von der Aminosäureposition Nummer 17 bis zur Aminosäureposition Nummer 57 in Tabelle A reichende 41 Aminosäuren lange Sequenz enthält.

**15.** Baculovirus nach Anspruch 14, worin die intronfreie DNA Sequenz ein Polypeptid kodiert, das die von der Aminosäureposition Nummer 17 bis zur Aminosäureposition Nummer 58 in Tabelle A reichende 42 Aminosäuren lange Sequenz enthält.

**16.** Baculovirus DNA nach Anspruch 15, worin die intronfreie DNA Sequenz für ein Polypeptid kodiert, das die von der Aminosäureposition 1 zur Aminosäureposition 58 in Tabelle A reichenden 58 Aminosäuren enthält.

**17.** Baculovirus nach Anspruch 14, das Bombyx mori NPV DNA oder Autographa californica NPV DNA ist.

**18.** Insektizide Zusammensetzung, die eine insektizid wirksame Menge eines Hybridbaculovirus nach den Ansprüchen 12-17 und einen inerten Träger enthält.

**19.** Verfahren zur Bekämpfung von Insekten, die gegenüber einem NPV Baculovirus empfindlich sind, gekennzeichnet durch Anwenden einer insektizid wirksamen Menge einer Hybrid-Baculovirus DNA nach den Ansprüchen 12-17 in einem inerten Träger am Verbreitungsort der Insekten.

**Revendications**

**1.** Polypeptide qui a l'activité d'un facteur diurétique chez les insectes, essentiellement exempt de polypeptides d'insecte associés, comportant un carbone terminal amidé et une séquence d' acides aminés selon la formule (I)

```
Arg-Met-Pro-Ser-Leu-Ser-Ile-Asp-Leu-Pro
Met-Ser-Val-Leu-Arg-Gln-Lys-Leu-Ser-Leu    (formule  I)
Glu-Lys-Glu-Arg-Lys-Val-His-Ala-Leu-Arg
Ala-Ala-Ala-Asn-Arg-Asn-Phe-Leu-Asn-Asp
Ile-NH2
```

ou un analogue ou un dérivé du polypeptide de formule (I), où un ou plusieurs résidus d'acides aminés ont été supprimés, ou ajoutés, ou substitués par un résidu d' acide aminé différent, sans réduction sensible de l'activité diurétique du peptide.

2. Polypetide précurseur qui a l'activité d'un facteur diurétique chez les insectes, essentiellement exempt de polypeptides d'insecte associés, comportant une séquence d'acides aminés selon la formule (II)

```
Arg-Met-Pro-Ser-Leu-Ser-Ile-Asp-Leu-Pro
Met-Ser-Val-Leu-Arg-Gln-Lys-Leu-Ser-Leu    (formule  II)
Glu-Lys-Glu-Arg-Lys-Val-His-Ala-Leu-Arg
Ala-Ala-Ala-Asn-Arg-Asn-Phe-Leu-Asn-Asp
Ile-X
```

où X est Gly, Gly-Lys ou Gly-Lys-Arg,
ou un analogue ou un dérivé du polypeptide de formule (II), où un ou plusieurs résidus d'acides aminés ont été supprimés, ou ajoutés, ou substitués par un résidu d'acide aminé différent, sans réduction sensible de l'activité diurétique du peptide.

3. Polypeptide ayant une activité diurétique pour les insectes, codé a) par une séquence d'ADN comprenant les 123 nucléotides s'étendant de la position de nucléotide 54 à la position de nucléotide 177 dans le Tableau A; ou b) par une séquence nucléotidique qui s'hybride dans des conditions sévères sur cette séquence de 123 nucléotides.

4. ADN isolé comprenant une séquence d'ADN sans intron codant pour un polypeptide comprenant la séquence de 41 acides aminés s'étendant de la position d'acide aminé 17 à la position d'acide aminé 57 dans le Tableau A.

5. ADN selon la revendication 4, dans lequel la séquence d'ADN sans intron code pour un polypeptide comprenant la séquence de 42 acides aminés s'étendant de la position d'acide aminé 17 à la position d'acide aminé 58 dans le Tableau A.

6. ADN vecteur comprenant une séquence d'ADN codant pour un polypeptide selon l'une quelconque des revendications 1 à 3.

7. ADN vecteur selon la revendication 6, dans lequel la séquence d'ADN subit un contrôle d'expressin de l'ADN promoteur hétérologue, ladite séquence d'ADN comprenant une séquence d'ADN sans intron codant pour le polypeptide qui comprend la séquence de 41 acides aminés s'étendant de la position d'acide aminé 17 à la position d'acide aminé 57 dans le Tableau A.

8. ADN vecteur selon la revendication 7, dans lequel la séquence d'ADN sans intron code pour un polypeptide qui comprend la séquence de 42 acides aminés s'étendant de la position d'acide aminé 17 à la position d'acide aminé 58 dans le Tableau A.

**9.** ADN vecteur selon la revendication 7, dans lequel l'ADN promoteur est un promoteur de la polyédrine de NPV.

**10.** ADN vecteur selon la revendication 9, dans lequel ladite séquence d'ADN codant pour le polypeplide est flanquée, en position 5' et 3', par une séquence d'ADN de virus d'insecte comprenant l'ADN du gène structural de la polyédrine du virus d'insecte.

**11.** ADN vecteur selon la revendication 9, dans lequel le promoteur est un promoteur de la polyédrine de Bombyx mori ou un promoteur de la polyédrine d'Autographa californica.

**12.** ADN de baculovirus hybride pouvant conduire à une infection létale de cellules d'insecte et comprenant un ADN de baculovirus et une séquence d'ADN codant pour un polypeptide selon l'une quelconque des revendications 1-3, ladite séquence codante étant soumise au contrôle d'expression d'un promoteur pouvant effectuer l'expression de ladite séquence codante dans le baculovirus.

**13.** ADN de baculovirus selon la revendication 12, dans lequel ladite séquence d'ADN codant pour le polypeptide comprend : a) les 123 nucléotides s'étendant de la position de nucléotide 54 à la position de nucléotide 177 dans le Tableau A); ou b) une séquence nucléotidique qui s'hybride dans des conditions d'hydridation sévères sur une telle séquence de 123 nucléotides.

**14.** ADN de baculovirus selon la revendication 12, dans lequel ladite séquence d'ADN codant pour le polypeptide comprend une séquence d'ADN sans intron codant pour le polypeptide qui comprend la séquence de 41 acides aminés s'étendant de la position d'acide aminé 17 à la position d'acide aminé 57 dans le Tableau A.

**15.** Baculovirus selon la revendication 14, dans lequel la séquence d'ADN sans intron code pour un polypeptide comprenant la séquence de 42 acides aminés qui s'étend de la position d'acide aminé 17 à la position d'acide aminé 58 dans le Tableau A.

**16.** Baculovirus selon la revendication 15, dans lequel la séquence d'ADN sans intron code pour un polypeptide qui comprend les 58 acides aminés s'étendant de la position d'acide aminé 1 à la position d'acide aminé 58 dans le Tableau A.

**17.** Baculovirus selon la revendication 14, qui est l'ADN du NPV de Bombyx mori ou l'ADN du NPV d'Autographa californica.

**18.** Composition insecticide comprenant une quantité à effet insecticide d'un baculovirus hybride selon les revendications 12 à 17 et un support inerte.

**19.** Procédé pour combattre les insectes sensibles à un baculovirus NPV, qui comprend l'application sur l'habitat des insectes d'une quantité à effet insecticide d'un ADN de baculovirus hybride selon les revendications 12-17 dans un support inerte.

EP 0 359 714 B1

Fig. 1

```
        -10            1              10             20             730
         *             *              *              *              *
                      MetProAsnTyrSerTyrThr  ...ProAlaTyr -
        ..AAAACCTATAAATATGCCGAATTATTCATACACC  ...CCGGCGTATTAAAA...
```

Fig. 2

```
              -10  -5                                           740
               *    *                  XbaI                      *
pBE284...........AAAACCTATAAggaattctagataggcctTAAAACAC..
                                EcoRI           AatI
```

Fig. 3

29

Fig. 4